# EUROPEAN PATENT APPLICATION

(11) **EP 1 576 985 A1**
(43) Date of publication of application: **21.09.2005**
(21) Application number: 04447066.4
(22) Date of filing: 18.03.2004
(51) Int. Cl.: A61P 25/28, A61K 31/4545, A61K 31/00

(54) **Use of D4 and 5-HT2A antagonists, inverse agonists or partial agonists**

(71) Applicant: B&B Beheer NV, 3570 Alken (BE)
(72) Inventor: Buntinx, Erik, 3570 Alken (BE)
(74) Representative: De Clercq, Ann G. Y.

(57) **Abstract**

The present invention relates to the use of compounds and compositions of compounds having D4 and/or 5-HT2A antagonistic, partial agonistic or inverse agonistic activity in combination with other compounds for treating neurodegenerative diseases or disorders, such as Parkinson disease and related cognitive diseases or disorders. The invention also relates to pharmaceutical compositions for administration to a patient diagnosed as having a neurodegenerative disease or disorder and/or a cognitive disease or disorder, said pharmaceutical composition containing (**i**) compounds having D4 antagonistic, partial agonistic or inverse agonistic activity and/or (ii) compounds having 5-HT2A antagonistic, partial agonistic or inverse agonistic, and/or (iii) any known medicinal compound or compositions of said compounds in combination with another compound for treating said diseases or disorders to augment the therapeutic effect or to provide a faster onset of the therapeutic effect of said other compound.

## Description

### Field of the invention

The invention relates to the field of neuro-degenerative diseases and related cognitive diseases or disorders. More specifically, the invention relates to the use of compounds which have D4 and/or 5-HT2A antagonist, inverse agonist or partial agonist activity for the preparation of medicaments.

### Background to the invention

Data demonstrate that dopamine D4 receptors play an important role in the induction of behavioral sensitization to amphetamine and accompanying adaptations in pre- and postsynaptic neural systems associated with the meso-limbo-cortical dopamine projections (D. L. Feldpausch et al; The journal of pharmacology and experimental therapeutics Vol. 286, Issue 1, 497-508, July 1998). Further, results show that dopamine D4 receptor antagonism in the brain does not result in the same neuro-chemical consequences (increased dopamine metabolism or hyperprolactinemia) observed with typical neuroleptics (Smita Patel et al; The journal of pharmacology and experimental therapeutics Vol. 283, Issue 2, 636-647, 1997). The selective D4 dopamine receptor antagonist L-745,870 was ineffective as an antipsychotic for the treatment of neuroleptic responsive inpatients with acute schizophrenia (Kramer MS et al; Arch Gen Psychiatry 1997 Dec; 54(12):1080).

It has been shown that dopamine D4 receptors play an important role in novelty-seeking and exploratory behaviors mediated by dopamine in the basal ganglia. Furthermore, electron microscopy studies showed the exclusive presynaptic localization of D4 receptors in the two main striatal output fields, the globus pallidus and the substantia nigra pars reticulata. In contrast, double labeling experiments using immunofluorescent secondary antibodies showed that choline acetyltransferase-, somatostatin- and parvalbumin-containing interneurons did not express D4 receptors (Cuéllar, B., Rivera, A., Martin, A.B.,¹ De la Calle, A. and Moratalla, R. - Facultad de Biología. Dpto. Biología Celular. Universidad de Málaga).

Direct-acting dopamine agonists - important in the treatment of Parkinson Disease because they relieve the excessive burden of remaining neurons without being subject to metabolism into toxic free radicals inside dopamine neurons as has been hypothesized for levodopa - have significant drawbacks and side effects. Their potency is lower than that of levodopa and they can rarely be used as mono-therapy, except in the initial stages of the disease. As ergoline derivatives, they are not very specific and interact with several subtypes of dopamine receptors as well as with 5-HT receptors. D1 stimulation may contribute to the occurrence of dyskinesias, while 5-HT and D4 stimulation may result in confusion, hallucinations and other psychiatric manifestations. Dopamine agonists act also at the periphery, and this may contribute to significant side effects such as orthostatic hypotension and nausea. (Amos D. Korczyn).

Moreover, in the nigrostriatal dopaminergic neuron serotonin inhibits dopamine release via its 5-HT2A receptor, both at the level of dopamine cell bodies in the brainstem substantia nigra and at the level of the axon terminals in the basal ganglia-neostriatum. In both cases, the release of serotonin acts as a "brake" on dopamine release (S.M. Stahl, Essential Psychopharmacology).

Post mortem and in vivo studies have revealed a loss of dopamine D2 receptors in the temporal lobes in Alzheimer Disease (AD). Moreover, the role of hippo-campal D2 receptors on memory performance has been suggested in experimental studies. Multiple linear regression analysis showed that the D2 receptor binding potentials in the right hippocampus had a significant positive association with verbal memory performance (Wechsler Memory Scale - Revised) (P = 0.001) and picture naming (the Boston Naming Test) (P = 0.002). The findings suggest a role for temporal lobe D2 receptors in the memory and naming performance in AD. (Kemppainen N, Laine M, Laakso MP, Kaasinen V, Någren K, Vahlberg T, Kurki T, Rinne JO.; Eur J Neurosci 2003;18:149-154). Atypical antipsychotic drugs (SDA's) may improve cognitive functions in both schizophrenic and Alzheimer patients. They may boost the actions of cholinesterase inhibitors in Alzheimer's disease throughout a blockade of the 5HT2A receptors in the mesocortical dopamine pathways leading to dopamine release, which could compensate for the dopamine deficiency caused by the neurotoxic process of the plaques, which are produced from β-amyloid protein (βA4). Otherwise, SDA's are blocking the postsynaptic dopamine 2 receptors by their high dopamine receptor D2 affinity merely in the mesolimbic pathway - and this will in fact at least partially counteract the mentioned boosting.

In addition, since Alzheimer disease obviously is accompanied with many behavioral disturbances - merely aggression and confusion - there is also very often a need for the synergistic actions between a cholinesterase inhibitor and a behavior regulating agent.

It is therefore an aim of the present invention to provide for new and effective therapies for treating neurodegenerative diseases such as Parkinson Disease and related cognitive diseases or disorders.

### Summary of the invention

The present invention relates to the use of compounds and pharmaceutical compositions having D4 and/or 5-HT2A antagonistic, partial agonistic or inverse agonistic activity for the preparation of medicaments and to methods entailing administering to a patient in need thereof a pharmaceutical composition containing (i) compounds having D4 antagonistic, partial agonistic or inverse agonistic activity and/or (ii) compounds having 5-HT2A antagonistic, partial agonistic or inverse agonistic, and/or (iii) any known medicinal compound or composition of compounds. The combined D4 and 5-HT2A antagonistic, partial agonistic or inverse agonistic effects may reside within a single compound or in two different compounds. In one embodiment, the term "first compound" relates to a compound having both D4 and 5-HT2A antagonistic, partial agonistic or inverse agonistic activity. In another embodiment, it is contemplated that the term "first composition" is used to indicate a composition comprising a compound having D4 antagonistic, partial agonistic or inverse agonistic activity and a compound having 5-HT2A antagonistic, partial agonistic or inverse agonistic activity.

5-HT2A antagonists, partial agonists or inverse agonists according to the invention are, for instance, pipamperone, fananserin, L-745,870, PNU-101387G and U-101387.

Preferably, the compounds of the invention which have a selective affinity for the 5-HT2A receptor, are compounds which have a pKi value equal to or higher than 8 towards the 5-HT2A receptor and less than 8 towards other 5HT receptors.

D4 antagonists, partial agonists or inverse agonists according to the invention are, for instance, pipamperone, fananserin, ORG 5222, zotepine, olanzepine, clozapine, S16924, S18327, amperozide, serindole, MDL 100.907, tiospirone, fluspirilene, ocaperidone, paliperidone, risperidone and ziprasidone.

Preferably, the compounds of the invention have a selective affinity for the D4 receptor , are compounds which have a pKi value equal to or higher than 8 towards the D4 receptor and less than 8 towards other Dopamine receptors.

One example of a compound which has both a selective affinity for the 5-HT2A receptor with a pKi value equal to or higher than 8 towards the 5-HT2A receptor and less than 8 towards other 5HT receptors, and a selective affinity for the D4 receptor with a pKi value equal to or higher than 8 towards the D4 receptor and less than 8 towards other Dopamine receptors and which is therefore useful in a combination therapy is pipamperone.

Further, the invention relates to the use of first compounds or compositions as defined above for treating a disease whereby a further, i.e. a second or third, compound is administered simultaneously with, separate from or sequential to said first compound or composition to augment the therapeutic effect of said further compound on said disease, or to provide a faster onset of the therapeutic effect of said further compound on said disease.

According to a first embodiment, the present invention relates to the use of a first compound for the preparation of a medicament for treating neurodegenerative diseases or disorders and/or (related) cognitive diseases or disorders, characterized in that said compound is administered simultaneously with, separate from or prior to the administration of a second compound to augment the therapeutic effect or to provide a faster onset of the therapeutic effect of said second compound, further characterized in that said first compound has (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors. Preferably, said first compound is pipamperone, preferably to said first compound is to be administered to a patient in a dose ranging between 5 and 15 mg of the active ingredient.

More particularly, the invention relates to any of the uses described above, wherein said neurodegenerative disease or disorder is Parkinson Disease.

The present invention further relates to any of the uses described above, wherein said first compound is to be administered daily at least one day before administering said second compound.

According to a preferred embodiment, the present invention relates to any of the uses described above, wherein said second compound is a dopamine receptor agonist. Preferably, said dopamine receptor agonist is chosen from the group consisting of amantadine, bromocriptine, cabergoline lisuride, pergolide, ropinirole and pramipexole, or a pro-drug or an active metabolite thereof, or a pharmaceutically acceptable salt thereof. More preferably, said dopamine receptor agonist is pergolide and is to be administered in a dose ranging between 0.5 and 10 mg of the active ingredient.

In particular, the invention also relates to a pharmaceutical composition comprising
(a) a compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors and
(b) a dopamine receptor agonist,
   as a combined preparation for simultaneous, separate or sequential use for treating a neurodegenerative disease or disorder such as Parkinson Disease.

According to another preferred embodiment, the present invention relates to any of the uses described above, wherein said second compound is levodopa associated with a decarboxylase inhibitor. Preferably, said levodopa/decarboxylase-inhibitor is chosen from the group consisting of levodopa/carbidopa and levodopa/benserazide, or a pro-drug or an active metabolite thereof, or a pharmaceutically acceptable salt thereof. More preferably, said levodopa/decarboxylase-inhibitor is levodopa/carbidopa and is to be administered in a dose ranging between 2000 mg/200 mg and 100 mg/10 mg of the active ingredients.

In particular, the invention also relates to a pharmaceutical composition comprising
(a) a compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors, and
(b) a levodopa associated with a decarboxylase inhibitor,
   as a combined preparation for simultaneous, separate or sequential use for treating a neurodegenerative disease or disorder such as Parkinson Disease.

According to a further preferred embodiment, the present invention relates to any of the uses described above, wherein said second compound is a mono-amine oxidase B (MAO-B) inhibitor. Preferably, said second compound is selegilinehydrochloride or a pro-drug or an active metabolite thereof, or a pharmaceutically acceptable salt thereof. More preferably, said selegilinehydrochloride is to be administered in a dose ranging between 2 and 25 mg of the active ingredient.

In particular, the invention also relates to a pharmaceutical composition comprising
(a) a compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors and
(b) a mono-amine oxidase B (MAO-B) inhibitor,
   as a combined preparation for simultaneous, separate or sequential use for treating a neurodegenerative disease or disorder such as Parkinson Disease.

The present invention further relates to the use of a composition for the preparation of a medicament for treating a neurodegenerative disease or disorder and/or a (related) cognitive disease or disorder, characterized in that said composition is administered simultaneously with, separate from or prior to the administration of a third compound to augment the therapeutic effect or to provide a faster onset of the therapeutic effect of said third compound, further characterized in that said composition comprises a first compound having (i) a selective affinity for the D4 receptor with a pKi value equal to or higher than 8 towards the D4 receptor and less than 8 towards other Dopamine receptors, and a second compound having (ii) a selective affinity for the 5-HT2A receptor with a pKi value equal to or higher than 8 towards the 5-HT2A receptor and less than 8 towards other 5HT receptors. According to a preferred embodiment, said neurodegenerative disease or disorder is Parkinson Disease.

In particular, the invention relates to the use described above, wherein said first compound is chosen from the group consisting of, pipamperone, fananserin, L-745,870, PNU-101387G and U-101387 or a pro-drug or a pharmaceutically acceptable salt thereof and wherein said second compound is chosen from the group comprising pipamperone, fananserin, ORG 5222, zotepine, olanzepine, clozapine, S16924, S18327, amperozide, serindole, MDL 100.907, tiospirone, fluspirilene, ocaperidone, risperidone, paliperidone and ziprasidone or a pro-drug or an active metabolite thereof, or a pharmaceutically acceptable salt thereof. Preferably, the composition referred to in the above uses is to be administered to a patient in a dose ranging between 0,5 µg and 2000 mg for each of the active ingredients.

According to a preferred embodiment, the invention relates to any of the uses described above, wherein said third compound is a dopamine receptor agonist. Preferably said dopamine receptor agonist is chosen from the group consisting of amantadine, bromocriptine, cabergoline lisuride, pergolide, ropinirole and pramipexole, or a pro-drug or an active metabolite thereof, or a pharmaceutically acceptable salt thereof. More preferably, said dopamine receptor agonist is pergolide and is to be administered in a dose ranging between 0.5 and 10 mg of the active ingredient.

In particular, the present invention relates to a pharmaceutical composition comprising
(a) a compound having a selective affinity for the Dopamine-4 (D4) receptor with a pKi value equal to or higher than 8 towards the D4 receptor and less than 8 towards other Dopamine receptors,
(b) a compound having a selective affinity for the 5-HT2A receptor with a pKi value equal to or higher than 8 towards the 5-HT2A receptor and less than 8 towards other 5HT receptors, and
(c) a dopamine receptor agonist,
   as a combined preparation for simultaneous, separate or sequential use for treating a neurodegenerative disease or disorder such as Parkinson Disease.

According to another preferred embodiment, the invention relates to any of the uses described above, wherein said third compound is levodopa associated with a decarboxylase inhibitor. Preferably, said levodopa/decarboxylase-inhibitor is chosen from the group comprising levodopa/carbidopa, levodopa/benserazide, or a pro-drug or an active metabolite thereof, or a pharmaceutically acceptable salt thereof. More preferably, said levodopa/decarboxylase-inhibitor is levodopa/carbidopa and is to be administered in a dose ranging between 2000 mg/200 mg and 100 mg/10 mg of the active ingredients.

In particular, the present invention relates to a pharmaceutical composition comprising
(a) a compound having a selective affinity for the Dopamine-4 (D4) receptor with a pKi value equal to or higher than 8 towards the D4 receptor and less than 8 towards other Dopamine receptors, and
(b) a compound having a selective affinity for the 5-HT2A receptor with a pKi value equal to or higher than 8 towards the 5-HT2A receptor and less than 8 towards other 5HT receptors, and
(c) levodopa associated with a decarboxylase inhibitor,
   as a combined preparation for simultaneous, separate or sequential use for treating a neurodegenerative disease or disorder such as Parkinson Disease.

According to a further preferred embodiment, the invention relates to any of the uses described above, wherein said third compound is a mono-amine oxidase B (MAO-B) inhibitor. Preferably, said mono-amine oxidase B (MAO-B) inhibitor is selegelinehydrochloride or a pro-drug or an active metabolite thereof, or a pharmaceutically acceptable salt thereof. More preferably, said selegilinehydrochloride is to be administered in a dose ranging between 2 and 25 mg of the active ingredient.

In particular, the present invention relates to a pharmaceutical composition comprising
(a) a compound having a selective affinity for the Dopamine-4 (D4) receptor with a pKi value equal to or higher than 8 towards the D4 receptor and less than 8 towards other Dopamine receptors, and
(b) a compound having selective affinity for the 5-HT2A receptor with a pKi value equal to or higher than 8 towards the 5-HT2A receptor and less than 8 towards other 5HT receptors. and
(c) a mono-amine oxidase B (MAO-B) inhibitor,
   as a combined preparation for simultaneous, separate or sequential use for treating a neurodegenerative disease or disorder such as Parkinson Disease.

According to a further embodiment, the present invention relates to the use of a first compound or a composition for the preparation of a medicament for treating a cognitive disease or disorder, characterized in that said compound or composition is administered simultaneously with, separate from or prior to the administration of a further compound to augment the therapeutic effect or to provide a faster onset of the therapeutic effect of said further compound, further characterized in that said first compound has (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors; or characterized in that said composition comprises a first compound having (i) a selective affinity for the D4 receptor with a pKi value equal to or higher than 8 towards the D4 receptor and less than 8 towards other Dopamine receptors, and a second compound having (ii) a selective affinity for the 5-HT2A receptor with a pKi value equal to or higher than 8 towards the 5-HT2A receptor and less than 8 towards other 5HT receptors.

The invention also relates to the use of a compound as defined above, or of a composition as defined above, for the preparation of a medicament for treating a cognitive disease or disorder, characterized in that said compound or composition is administered simultaneously with, separate from or sequential to a cholinesterase inhibitor to augment the therapeutic effect or to provide a faster onset of the therapeutic effect of said cholinesterase inhibitor. Preferably, said disease or disorder is selected from the group consisting of delirium; dementia, such as Alzheimer Disease, substance-induced persisting dementia, vascular dementia, dementia due to a general medical condition chosen from the group comprising HIV disease, head trauma, Parkinson Disease, Huntington Disease, Pick Disease and Creutzfeldt-Jacob Disease; amnestic disorders due to a general medical condition or a substance-induced persisting amnestic disorder; mild cognitive impairment disorder; and other cognitive disorders.

The invention further relates to the any of the uses described above, wherein said cholinesterase inhibitor is chosen from the group consisting of donepezil, ENA-713, galantamine, memantine and tacrine, or a pro-drug or an active metabolite thereof, or a pharmaceutically acceptable salt thereof. Preferably said cholinesterase inhibitor is galantamine and is to be administered in a dose ranging between 5 and 50 mg of the active ingredient.

In particular, the present invention relates to a pharmaceutical composition comprising
(a) a compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors, and
(b) a cholinesterase inhibitor
   as a combined preparation for simultaneous, separate or sequential use for treating a cognitive disease or disorder.

The invention also particularly relates to a pharmaceutical composition comprising
(a) a compound having a selective affinity for the Dopamine-4 (D4) receptor with a pKi value equal to or higher than 8 towards the D4 receptor and less than 8 towards other Dopamine receptors, and
(b) a compound having a selective affinity for the 5-HT2A receptor with a pKi value equal to or higher than 8 towards the 5-HT2A receptor and less than 8 towards other 5HT receptors, and
(c) a cholinesterase inhibitor,
   as a combined preparation for simultaneous, separate or sequential use for treating a cognitive disease or disorder.

The invention further relates to any of the pharmaceutical composition herein described wherein said compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors, is pipamperone and is present in the composition in a dose ranging between 5 and 15 mg of active ingredient, expressed as the daily dose to be administered to a patient in need thereof.

### Detailed description of the invention

The present inventors surprisingly found that compounds which have a high selective affinity towards the 5-HT2A receptor and which, at the same time have a high selective affinity towards the Dopamine-4 (D4) receptor show an improved effect in combination with other compounds when treating certain diseases or disorders.

The combined D4 and 5-HT2A antagonistic, partial agonistic or inverse agonistic effects may reside within the same chemical or biological compound or in two different chemical or two different biological compounds or in a combination of a chemical & biological compound.

In one embodiment of the invention, the 5-HT2A receptor and/or Dopamine-4 receptor antagonist, inverse agonist or partial agonist is used in treatment of patients having neurodegenerative diseases or disorders, or related cognitive diseases or disorders. The diseases or disorders of the present invention are characterized by an underlying degeneration of the Central Nervous System (CNS), preferably selected from the group consisting of, but not limited to, neurodegenerative diseases such as Parkinson Disease, and related cognitive diseases or disorders such as Alzheimer Disease.

Parkinson Disease, which is a chronic progressive nervous disease chiefly of later life, is linked to decreased dopamine production in the substantia nigra and is marked by tremor and weakness of resting muscles and by a shuffling gait. Because of the need of specific D4 and 5-HT2A antagonism in the treatment of Parkinson Disease with Dopamine agonists and even levodopa, it seems reasonable to combine with a compound with a high selective D4 and 5-HT2A antagonism i.e. merely no activity towards the other receptors especially the D2 receptor because of the primary need of the relieve of the excessive burden of remaining Dopaminergic neurons. Therefore, the use of the so called atypical anti-psychotics or serotonin-dopamine antagonists (SDA's) are absolutely contra-indicated since their high affinity for the D2 receptor. Even the use of serotonin releasing compounds such as SSRI's in the absence of an effective 5-HT2A antagonism are contra-productive towards the Parkinson symptoms although many Parkinson patients are in need for an antidepressant since major depression is a very common and disabling condition in this kind of patients.

The expression "(related) cognitive diseases or disorders" according to the invention comprises, the following group of diseases or disorders: delirium (F05), dementia (such as Alzheimer Disease (F00), vascular dementia (F01), dementia due to other general medical conditions (HIV disease (F02.4), head trauma (F06.8), Parkinson Disease (F02.3), Huntington Disease (F02.2), Pick Disease (F02.0), Creutzfeldt-Jacob Disease (F02.1) and other (F02.8)), substance-induced persisting dementia (F1x.6)), amnestic disorders due to a general medical condition (F06.8) or a substance-induced persisting amnestic disorder (F1x.6), mild cognitive impairment disorder (F06.7) and other cognitive disorders (F04). The above list of diseases is provided by way of example and is not intended to limit the invention.

Alzheimer Disease is a degenerative brain disease of unknown cause that is the most common form of dementia. Alzheimer Disease usually starts in late middle age or in old age as a memory loss for recent events spreading to memories for more distant events and progresses over the course of five to ten years to a profound intellectual decline characterized by dementia and personal helplessness. The disease is marked histologically by the degeneration of brain neurons especially in the cerebral cortex and by the presence of neurofibrillary tangles and plaques containing beta-amyloid. Because dopamine receptor D4 (DRD4) antagonism can inhibit the behavioral disturbances - merely aggression and confusion - accompanied with Alzheimer disease and 5HT2A antagonism has an important boosting effect towards the effect of cholinesterase inhibitors like used in the treatment, a high selective D4/5HT2A-antagonist would be a more preferable compound to combine with a cholinesterase inhibitor since this avoids the counteracting effect of SDA's on the cognitive functioning by its dopamine receptor D2-antagonism.

These diseases and their diagnosis are very clearly defined in the *"International Statistical Classification of Diseases and Related Health Problems, 1989 Revision, Geneva, World Health Organization, 1992 (ICD-10).* This manual sets forth diagnostic criteria, descriptions and other information to guide the classification and diagnosis of neurodegenerative disorders and is commonly used in the field of neurology. According to the ICD-10 classification, the cognitive disorders are classified under several classes of disorders, i.e. dispersed under categories F00 to F19 (see above: respective classification between parentheses). Following the DSM classification, however, they are grouped in one class of diseases or disorders. The "*Diagnostic and Statistical Manual of Mental Disorders* (DSM)" is published by the American Psychiatric Association. This manual sets forth diagnostic criteria, descriptions and other information to guide the classification and diagnosis of mental disorders and is commonly used in the field of neuropsychiatry. It is for instance available on the internet under: http://www.behavenet.com/capsules/disorders/ dsm4tr.htm.

A problem to be solved by the present invention is thus the provision of a more efficient therapy and efficient, highly selective and efficacious medicaments for treating neurodegenerative disorders and/or related cognitive disorders.

The inventors found that the non-response to dopamine receptor agonists (DA's) in Parkinson Disease may be declared by (partial) inhibition of the dopamine release since in the nigrostriatal dopaminergic neuron serotonin inhibits dopamine release via its 5-HT2A receptor stimulation. Des-inhibition thereof via 5-HT2A antagonism seems to be an answer to this problem.

The present inventors found that a simultaneous or foregoing treatment of the patient with a compound having a high selective 5-HT2A antagonist, inverse agonist or partial agonist activity, could lead to a greater response towards dopamine receptor agonists. However, not all compounds exhibiting 5-HT2A antagonism are useful: competition between 5-HT2A stimulation via serotonin and 5-HT2A antagonism via the compound could be responsible for the lack of more efficacy of compounds which have both a selective serotonin re-uptake inhibitory and 5-HT2A antagonist profile, such as trazodone and nefazodone.

The present inventors further surprisingly found that a simultaneous or foregoing treatment with a compound having a high selective D4 antagonist, inverse agonist or partial agonist activity in combination with a compound having a high selective 5-HT2A antagonist, inverse agonist or partial agonist activity could lead to a greater response not only towards dopamine receptor agonists but also towards other compounds such as, but not limited to, levodopa/decarboxylase-inhibitors, mono-amine-oxidase B inhibitors and cholinesterase inhibitors.

The present inventors found that a compound which binds to the 5-HT2A receptor with a pKi of at least 8 but for which the binding affinity, ie pKi, towards other 5HT receptors is less than 8 in combination with a compound which has a high selective affinity for the D4 receptor, i.e. which bind to the D4 receptor with a pKi of at least 8 but for which the binding affinity, ie pKi, towards other dopamine receptors is less than 8 also show such an improved effect in treatment. These effects, ie D4 antagonism, inverse agonism or partial agonism and 5-HT2A antagonism, inverse agonism or partial agonism, preferably reside in the same compound. In other embodiments of the invention, these effects reside in separate compounds.

'Other 5HT receptors' as used herein are for instance 5-HT1 receptors (i.e. 5-HT1A, 5-HT1 B, 5-HT1D, 5-HT1 E, 5-HT1 F), 5-HT2B, 5-HT2C, 5-HT6 (rat) and 5-HT7 (rat).

5-HT2A responsive compounds according to the invention are, for instance pipamperone, fananserin, L-745,870, PNU-101387G and U-101387. All these compounds are known in the art and may be used in doses according to the supplier's or physician's prescription.

By the expression 'selective affinity for the 5-HT2A receptor' is meant that the receptor has a higher affinity for the 5-HT2A receptor than for other known 5-HT receptors, preferably having a pKi value equal to or higher than 8 towards the 5-HT2A receptor and less than 8 towards other known 5-HT receptors.

Table 1 illustrates the selective affinities of some compounds for several receptors, including 5-HT receptors. It should be noted that this table comprises a non-exhaustive list of exemplified compounds of the invention. The man skilled in the art will appreciate that this table can be easily completed with the pKi values for the other compounds mentioned in the present application, by using conventional methods for measuring pKi values, such as the methods described further.

Preferably, the compounds of the invention which have a selective affinity for the 5-HT2A receptor, are compounds which have a pKi value equal to or higher than 8 towards the 5-HT2A receptor and less than 8 towards other 5HT receptors, as can be measured, for instance by methods known in the art. For instance, the "NIMH Psychoactive Drug Screening Program (PDSP)" Kᵢ database (http://kidb.cwru.edu/nimh/5htp.php), is a unique resource in the public domain which provides information on the abilities of drugs to interact with an expanding number of molecular targets. The PDSP Kᵢ database serves as a data warehouse for published and internally-derived pKi, or affinity, values for a large number of drugs and drug candidates at an expanding number of G-protein coupled receptors, ion channels, transporters and enzymes. The PDSP internet site also provides for commonly used protocols and assays for measuring pKi values of 5HT receptors.

Preferred compounds which have a selective affinity for the 5-HT2A receptor according to the invention are, for instance amperozide and pipamperone.

The expression 'selective affinity for the D4 receptor' means that the receptor has a higher affinity for the Dopamine D4 receptor than for other known Dopamine receptors.

D4 responsive compounds according to the invention are, for instance, pipamperone, fananserin, ORG 5222, zotepine, olanzepine, clozapine, S16924, S18327, amperozide, serindole, MDL 100.907, tiospirone, fluspirilene, ocaperidone, risperidone, paliperidone and ziprasidone. All these compounds are known in the art and are to be used in doses according to the supplier's or physician's prescription.

'Other Dopamine receptors' are, for instance, D1, D2 and D3.

pKi values of test compounds for Dopamine receptors can be measured using commonly known assays (see above).

Compounds which have a selective affinity for the D4 receptor preferably have a pKi value equal to or higher than 8 towards the D4 receptor and less than 8 towards other known Dopamine receptors.

One example of a compound which has both a selective affinity for the 5-HT2A receptor with a pKi value equal to or higher than 8 towards the 5-HT2A receptor and less than 8 towards other 5HT receptors, and a selective affinity for the D4 receptor with a pKi value equal to or higher than 8 towards the D4 receptor and less than 8 towards other Dopamine receptors and which is therefore useful in a combination therapy is pipamperone. Pipamperone is the conventional name given for the compound of the formula 1'-[3-(p-Fluorobenzoyl)propyl]-[1,4'-bipiperidine]-4'-carboxamide. Pipamperone is also the active ingredient of dipiperon (Janssen, Cilag B.V).

Table 1 illustrates the selective affinity of for instance pipamperone for the 5-HT2A and for the D4 receptor. Table 1 further illustrates the low or absence of affinity of pipamperone for other receptors such as the adrenergic receptors Alpha 1A, Alpha 2A, Alpha 2B, Alpha 2C, Beta1, Beta2, and the histamine receptor H1. As such, treating patients with pipamperone will provide for less side effects which otherwise result from simultaneous stimulation of other receptors. Therefore, and according to preferred embodiments, useful compounds according to the invention not only have a selective 5-HT2A and/or D4 affinity but also a low affinity for other receptors such as the adrenergic and histamine receptors.

Further, the present inventors surprisingly found that the dosage of active ingredient for pipamperone in treatment could be very low compared to conventionally used dosages for treating mental disorders. Preferred dosages which, according to the invention, have been shown to be effective in combination therapy with other compounds, range between 5 and 15 mg per day or between 5 and 10 mg per day. More preferably, dosages of 5, 6, 7, 8, 9, 10, 11, 12, 13, 14 or 15 mg per day are used in treatment of the diseases of the invention. The present dosages are represented as unitary daily doses applicable for a person having an average (ie normal) body weight. It is appreciated that the man skilled in the art can knows how to adapt the unitary daily doses herein described when dealing with smaller or taller persons, or when dealing with children. Therefore, unitary daily doses can easily be converted, for instance to doses per kg of bodyweight. In conventional pipamperone treatment, the active ingredient is available in tablets of 40 mg per tablet or in solutions of 2 mg per drop. Conventional usage of high doses ranging from 40 to 360 mg is prescribed. For instance for children up to the age of 14, a doses corresponding with 2 to 6 mg per kg body weight is conventionally prescribed. The high selective affinity of pipamperone towards the 5-HT2A receptor and the D4 receptor is reflected in the low dosage which is needed for the treatment of the diseases or disorders listed herein and also contributes to the high selective affinity of the compound towards the 5-HT2A receptor and the D4 receptor and therefore also to the efficacy of the treatment.

In the following embodiments of the invention relating to treatment of neurodegenerative diseases or disorders or related cognitive diseases or disorders, treatment is preferably done in a combination therapy consisting of a simultaneous or foregoing treatment with pipamperone, said pipamperone preferably administered in a dose ranging from 5 to 15 mg of active ingredient per day.

According to a general aspect of the invention, it thus has been found by the present inventors that the compounds having a selective 5-HT2A and D4 antagonist, inverse agonist or partial agonist activity as described above are useful for augmenting the therapeutic effect of another, i.e. second compound on a disease.

According to another embodiment of the invention it has also been found that the compounds having a selective 5-HT2A and D4 antagonist, inverse agonist or partial agonist activity as described above are useful for providing a faster onset of the therapeutic effect of another, i.e. second compound on a disease.

One example of a neurodegenerative disorder which is treated using compounds having a high selective affinity for the 5-HT2A and D4 receptor, for instance pipamperone, in a combination therapy with a dopamine receptor agonist is for instance Parkinson Disease. It should however be clear that the term "neurodegenerative diseases" is not limited to the one disease exemplified herein.

The present inventors not only found that the selective 5-HT2A and D4 antagonists, inverse agonists or partial agonists have an effect in augmenting the therapeutic effect or in providing a faster onset of the therapeutic effect of dopamine receptors agonists, but that this effect is also obtained when treating neurodegenerative diseases or disorders, such as Parkinson Disease, with selective 5-HT2A and D4 antagonists, inverse agonists or partial agonists in combination with other pharmaceutical compounds. Some examples of other pharmaceutical compounds whose effects are augmented or where the onset of the effect is fastened upon simultaneous or fore-going treatment with a selective 5-HT2A and D4 antagonist, inverse agonist or partial agonist, are levodopa/decarboxylase inhibitors and mono-amine oxidase B (MAO-B) inhibitors.

The present inventors also found that the 5-HT2A and D4 antagonists, inverse agonists or partial agonists have similar effects on treatment or alleviating related cognitive diseases or disorders, such as Alzheimer Disease, with certain compounds, such as, but not limited to cholinesterase inhibitors.

The terms "treatment", "treating", and the like, as used herein include amelioration or elimination of a developed mental disease or condition once it has been established or alleviation of the characteristic symptoms of such disease or condition. As used herein these terms also encompass, depending on the condition of the patient, preventing the onset of a disease or condition or of symptoms associated with a disease or condition, including reducing the severity of a disease or condition or symptoms associated therewith prior to affliction with said disease or condition. Such prevention or reduction prior to affliction refers to administration of the compound or composition of the invention to a patient that is not at the time of administration afflicted with the disease or condition. "Preventing" also encompasses preventing the recurrence or relapse-prevention of a disease or condition or of symptoms associated therewith, for instance after a period of improvement. It should be clear that neurodegenerative conditions may be responsible for mental complaints. In this respect, the term "treating" also includes prevention of a mental disease or condition or amelioration or elimination of the developed mental disease or condition once it has been established or alleviation of the characteristic symptoms of such conditions.

As used herein, the term "medicament" also encompasses the terms "drug", "therapeutic", "potion" or other terms which are used in the field of medicine to indicate a preparation with therapeutic or prophylactic effect.

According to one embodiment, the invention thus relates to the use of a compound having a high selective affinity for the 5-HT2A and D4 receptor in combination with a dopamine receptor agonist, for instance chosen from the group consisting of, but not limited to amantadine, bromocriptine, cabergoline lisuride, pergolide, ropinirole and pramipexole, or a pro-drug or an active metabolite thereof, or a pharmaceutically acceptable salt thereof.

According to a preferred embodiment of the invention, the above described compounds having a 5-HT2A and D4 antagonist, inverse agonist or partial agonist activity are useful for augmenting the therapeutic effect of pergolide, or for providing a faster onset of the therapeutic effect of pergolide. Pergolide is a dopamine receptor agonist. Pergolide is administered in a dose ranging between 0.5 and 10 mg of the active ingredient per day. Preferably, daily doses of active ingredient ranging between 2 and 5 mg per day are administered. More preferably a daily dose of 3 mg of the active ingredient is administered.

According to a further embodiment, the invention relates to the use of pipamperone, preferably in a dose ranging from 5 to 15 mg of the active ingredient per day, in combination with pergolide, in a dose ranging from 0.5 to 10 mg of active ingredient per day, for treating Parkinson Disease.

The compound having a selective 5-HT2A and D4 antagonist, inverse agonist or partial agonist activity is thus also used in a combination therapy with other, i.e. second compounds in treatment of the same disease or disorder, i.e. neurodegenerative diseases such as Parkinson Disease.

In certain embodiments of the invention said second compound is levodopa associated with a decarboxylase-inhibitor. Preferred levodopa/decarboxylase-inhibitors to be administered to patients with an underlying degeneration of the CNS in combination with the selective 5-HT2A and D4 antagonist, inverse agonist or partial agonist of the invention are chosen from the group comprising, but not limited to levodopa/carbidopa, levodopa/benserazide, pharmaceutically acceptable salts, pro-drugs and mixtures thereof. These compounds are known in the art and may be used in doses according to the supplier's or physician's prescription.

According to a preferred embodiment of the invention, the above described compounds having a 5-HT2A and D4 antagonist, inverse agonist or partial agonist activity are useful for augmenting the therapeutic effect of levodopa/carbidopa, or for providing a faster onset of the therapeutic effect of levodopa/carbidopa.

Chemically, levodopa is known as (-)-3-(3,4-dihydroxy-phenyl)-L-alanine. It is commercially available for instance under the names dopar or larodopa. Levodopa is required by the brain to produce the neurotransmitter dopamine.

Levodopa/carbidopa is a combination drug comprising the active compound levodopa and the decarboxylase-inhibitor carbidopa: both compounds are administered in a combined preparation, i.e. the decarboxylase inhibitor prevents that levodopa is peripherally converted in dopamine, as such avoiding undesired peripheral dopamine activity; on the other hand, the decarboxylase does not pass the blood-brain barrier so that the inactive levodopa is converted in the CNS to the active dopa. Levodopa/carbidopa is commercially available for instance under the name sinemet (Merck & Co). Levodopa/carbidopa is administered in a dose ranging between 2000 mg and 100 mg (levodopa) and between 200 mg and 10 mg (carbidopa) per day, respectively for the active ingredients levodopa and carbidopa. As used herein, the doses of the combined levodopa/decarboxylase-inhibitor are noted: "between 2000 mg/ 200 mg and 100 mg/10 mg of the active ingredients". Preferably, daily doses of active ingredients ranging between 1500 mg/ 150 mg and 250 mg/ 25 mg per day are administered. More preferably a daily dose of 500 mg/50 mg of the active ingredients are administered.

According to a further embodiment, the invention relates to the use of pipamperone, preferably in a dose ranging from 5 to 15 mg of the active ingredient per day, in combination with levodopa/carbidopa, in a dose ranging from 2000 mg/ 200 mg to 100 mg/ 10 mg of active ingredients per day, for treating Parkinson Disease.

In other preferred embodiments, said second compound is a mono-amine oxidase B (MAO-B) inhibitor. Preferred MAO-B inhibitor to be administered to patients with an underlying degeneration of the CNS in combination with the selective 5-HT2A and D4 antagonist, inverse agonist or partial agonist of the invention are chosen from the group consisting of, but not limited to, selegelinehydrochloride, pharmaceutically acceptable salts, pro-drugs or active metabolites thereof, or mixtures thereof.

According to a preferred embodiment of the invention, the above described compounds having a 5-HT2A and D4 antagonist, inverse agonist or partial agonist activity are useful for augmenting the therapeutic effect of selegelinehydrochloride, or for providing a faster onset of the therapeutic effect of selegelinehydrochloride. Selegelinehydrochloride is a mono-amine oxidase B (MAO-B) inhibitor. Selegelinehydrochloride is administered in a dose ranging between 2 and 25 mg of the active ingredient per day. Preferably, daily doses of active ingredient ranging between 5 and 15 mg per day are administered. More preferably a daily dose of 7.5 mg of the active ingredient is administered.

According to a further embodiment, the invention relates to the use of pipamperone, preferably in a dose ranging from 5 to 15 mg of the active ingredient per day, in combination with selegelinehydrochloride, in a dose ranging from 0.5 to 10 mg of active ingredient per day, for treating Parkinson Disease.

In other embodiments of the invention, the second compound is used to treat another disease or group of diseases or disorders. In one such embodiment, said second compound is a cholinesterase inhibitor and is used for treating cognitive diseases, and the first compound, i.e. the selective 5-HT2A and D4 antagonist, inverse agonist or partial agonist of the invention, augments the therapeutic effect or provides for a faster onset of the therapeutic effect of said second compound on said cognitive disease or disorder.

The cognitive disease or disorder to be treated according to the combination therapy with a selective 5-HT2A and D4 antagonist, inverse agonist or partial agonist described above is selected from the group consisting of, but not limited to delirium, dementia (such as Alzheimer Disease, vascular dementia, dementia due to other general medical conditions (HIV disease, head trauma, Parkinson Disease, Huntington Disease, Pick Disease, Creutzfeldt-Jacob Disease and other), substance-induced persisting dementia), amnestic disorders due to a general medical condition or a substance-induced persisting amnestic disorder, mild cognitive impairment disorder and other cognitive disorders.

Preferred cholinesterase inhibitors to be administered in combination with the selective 5-HT2A and D4 antagonist, inverse agonist or partial agonist of the invention are chosen from the group consisting of, but not limited to, donepezil, ENA-713, galantamine, memantine and tacrine, or pharmaceutically acceptable salts thereof, or pro-drugs or active metabolites thereof, or mixtures thereof.

According to a preferred embodiment of the invention, the above described compounds having a 5-HT2A and D4 antagonist, inverse agonist or partial agonist activity are useful for augmenting the therapeutic effect of galantamine, or for providing a faster onset of the therapeutic effect of galantamine. Galantamine is a cholinesterase inhibitor. Galantamine is administered in a dose ranging between 5 and 50 mg of the active ingredient per day. Preferably, daily doses of active ingredient ranging between 10 and 30 mg per day are administered. More preferably a daily dose of 16 mg of the active ingredient is administered.

According to a further embodiment, the invention relates to the use of pipamperone, preferably in a dose ranging from 5 to 15 mg of the active ingredient per day, in combination with galantamine, in a dose ranging from 0.5 to 10 mg of active ingredient per day, for treating cognitive diseases or disorders, such as the ones described earlier.

From the above it should be clear that the selective 5-HT2A and D4 antagonist, inverse agonist or partial agonist is also named 'the first compound' in the embodiments of the invention.

According to the invention, when the 5-HT2A and D4 antagonist, inverse agonist or partial agonist activity reside in separate compounds, the term "composition" may be used. Compositions of the invention comprise a first compound having (i) a selective affinity for the D4 receptor with a pKi value equal to or higher than 8 towards the D4 receptor and less than 8 towards other Dopamine receptors, and a second compound having (ii) a selective affinity for the 5-HT2A receptor with a pKi value equal to or higher than 8 towards the 5-HT2A receptor and less than 8 towards other 5HT receptors.

The expression "the 5-HT2A and D4 antagonist, inverse agonist or partial agonist" is used herein to indicate a single compound having both activities or to indicate the composition comprising the activity in separate compounds.

It should be clear that when, in the present invention, a composition of separate compounds is used instead of a single compound, they may be used in combination with another, i.e. a third, compound to augment the therapeutic effect of the other, i.e. the third, compound on a disease.

When the 5-HT2A and D4 antagonist, inverse agonist or partial agonist and the second compound or third compound, are administered simultaneously, the compounds or active ingredients may be present in a single pharmaceutical composition or formulation. Alternatively the compounds or active ingredients are administered in separate pharmaceutical compositions or formulations for simultaneous or separate use.

When the 5-HT2A and D4 antagonist, inverse agonist or partial agonist of the invention is administered prior to the second or third compound, as defined, the 5-HT2A and D4 antagonist, inverse agonist or partial agonist is administered at least during 1 day prior to said second or third compound. Preferably the 5-HT2A and D4 antagonist, inverse agonist or partial agonist is administered for at least 1, 2 3, 4, 5, 6, 7, 8, 9 or 10 days, prior to the administration of the second or third compound. Preferably the 5-HT2A and D4 antagonist, inverse agonist or partial agonist is administered for at least 2, 3, 4, 5 weeks prior to the administration of the second or third compound, or for at least 1, 2, 3, 4, 5, 6, 7, 8, 9, 10, 11 or 12 months prior to the administration of the second or third compound.

Also encompassed by the invention are pro-drugs to these second or third compounds or active metabolites of these compounds.

The term "active metabolite" as used herein relates to a therapeutically active compound produced by the metabolism of a parent drug. Drugs administered to treat diseases are usually transformed (metabolized) within the body into a variety of related chemical forms (metabolites), some of which may have therapeutic activity (an active metabolite).

The present invention also encompasses the use of these second or third compounds, administered in the form of a pharmaceutically acceptable salt in admixture with a suitable pharmaceutically acceptable excipient.

The present invention in particular also relates to pharmaceutical compositions comprising the first and second and/or third compounds.

To prepare the pharmaceutical compositions of the invention, comprising the compounds or the combination of the first and second and/or third compounds described herein, an effective amount of the active ingredients, in acid or base addition salt form or base form, is combined in admixture with a pharmaceutically acceptable carrier, which can take a wide variety of forms depending on the form of preparation desired for administration. These pharmaceutical compositions are desirably in unitary dosage form suitable, for administration orally, nasal, rectally, percutaneously or by parenteral injection. Other preferred routes of administration according to the present invention are topical administration, for instance involving the use of transdermal administration such as transdermal patches or iontophoresis devices.

For example, in preparing the compositions in oral dosage form, any of the usual pharmaceutical inert media or diluents may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, solid dosage forms represent the most advantageous oral dosage unit form, in which case solid pharmaceutical carriers are obviously employed. Solid dosage forms for oral administration can include capsules, sustained-release capsules, tablets, sustained release tablets, chewable tablets, sublingual tablets, effervescent tablets, pills, powders, granules and gels. Such dosage forms can also comprise, as is normal practice, additional substances other than inert diluents, e.g., lubricating agents, such as magnesium stearate. In the case of capsules, tablets, effervescent tablets, and pills, the dosage forms can also comprise buffering agents. Soft gelatin capsules can be prepared to contain a mixture of the active compound or composition and vegetable oil. Hard gelatin capsules can contain granules of the active compound in combination with a solid, pulverulent carrier, such as lactose, saccharose, sorbitol, mannitol, potato starch, corn starch, amylopectin, or cellulose derivatives of gelatin. Tablets and pills can be prepared with enteric coatings.

"Sustained release" refers to the release of a therapeutically active compound and/or composition such that the blood levels of the therapeutically active compound are maintained within a desirable therapeutic range over an extended period of time. The sustained release formulation can be prepared using any conventional method known to one skilled in the art to obtain the desired release characteristics.

The compounds and compositions can also be applied topically using a transdermal system, such as one of an acrylic-based polymer adhesive with a resinous crosslinking agent impregnated with the composition and laminated to an impermeable backing. In a particular embodiment, the compositions of the present invention are administered as a transdermal patch, more particularly as a sustained-release transdermal patch. The transdermal patches of the present invention can include any conventional form such as, for example, adhesive matrix, polymeric matrix, reservoir patch, matrix or monolithic-type laminated structure, and are generally comprised of one or more backing layers, adhesives, penetration enhancers, an optional rate controlling membrane and a release liner which is removed to expose the adhesives prior to application. Polymeric matrix patches also comprise a polymeric-matrix forming material. Suitable transdermal patches are described in more detail in, for example, U.S. Pat. Nos. 5,262,165, 5,948,433, 6,010,715 and 6,071,531, the disclosure of each of which are incorporated herein in their entirety.

Another aspect of the present invention thus provides transdermal patches comprising a therapeutically effective amount of at least one of the compounds or compositions described herein to treat the diseases herein described, for instance one of the neurodegenerative diseases or disorders or related cognitive diseases or disorders herein specifically described. Preferably, in the combination therapies herein described, the 5HT2A and/or D4 antagonist, inverse agonist or partial agonist as defined above, and which preferably is pipamperone, and the second or third compounds, as herein described, are comprised within the same or distinct transdermal patch, said transdermal patches are for simultaneous, separate or sequential application on a patient's body.

Dosage forms for topical administration of the compounds and compositions can include creams, sprays, lotions, gels, ointments, and the like. In such dosage forms, the compositions of the invention can be mixed to form white, smooth, homogeneous, opaque cream or lotion with, for example, benzyl alcohol 1% or 2% (wt/wt) as a preservative, emulsifying wax, glycerin, isopropyl palmitate, lactic acid, purified water and sorbitol solution. In addition, the compositions can contain polyethylene glycol 400. They can be mixed to form ointments with, for example, benzyl alcohol 2% (wt/wt) as preservative, white petrolatum, emulsifying wax, and tenox II (butylated hydroxyanisole, propyl gallate, citric acid, propylene glycol). Woven pads or rolls of bandaging material, e.g., gauze, can be impregnated with the compositions in solution, lotion, cream, ointment or other such form can also be used for topical application. Liquid dosage forms for oral administration can include pharmaceutically acceptable emulsions, solutions, suspensions, syrups, and elixirs containing inert diluents commonly used in the art, such as water. Such compositions can also comprise adjuvants, such as wetting agents, emulsifying and suspending agents, and sweetening, flavoring, and perfuming agents.

Various delivery systems are known and can be used to administer the compounds or compositions of the present invention, including, for example, encapsulation in liposomes, microbubbles, emulsions, microparticles, microcapsules, nanoparticles, and the like. The required dosage can be administered as a single unit or in a sustained release form.

The term parenteral includes subcutaneous injections, intravenous, intramuscular, intrasternal injection, or infusion techniques. Injectable preparations, for example, sterile injectable aqueous or oleaginous suspensions can be formulated according to the known art using suitable dispersing agents, wetting agents and/or suspending agents. The sterile injectable preparation can also be a sterile injectable solution or suspension in a nontoxic parenterally acceptable diluent or solvent, for example, as a solution in 1,3-butanediol. Among the acceptable vehicles and solvents that can be used are water, Ringer's solution, and isotonic sodium chloride solution. Sterile fixed oils are also conventionally used as a solvent or suspending medium.

Examples of other pharmaceutical compounds whose effects are augmented or where the onset of the effect is fastened upon simultaneous or fore-going treatment with a selective 5-HT2A and D4 antagonist, inverse agonist or partial agonist as defined above, and which preferably is pipamperone according to a preferred embodiment of the invention, are: selective serotonin re-uptake inhibitors, nor-epinephrine re-uptake inhibitors, neuroleptic agents, and NK1 antagonists which all are used for treating diseases or disorders with an underlying dysregulation of the emotional functionality, for instance the mental disorders chosen from mood disorders, anxiety disorders, schizophrenia and other psychotic disorders, eating disorders, premenstrual syndrome, somatoform disorders, factitious disorders, dissociative disorders, sexual and gender identity disorders, sleep disorders, adjustment disorders, impulse control disorders, pervasive development, attention-deficit and disruptive behaviour disorders, substance-related disorders, personality disorders, psychological factors affecting medical conditions, malingering, antisocial behaviour, bereavement, occupational, identity, phase of life, academic problem, problems related to abuse or neglect.

Preferred selective serotonin re-uptake inhibitor are chosen from the group cconsisting of, but not limited to citalopram, fluoxetine, venlafaxine, fluvoxamine, paroxetine, sertraline, milnacipran and duloxetine, or a pro-drug or an active metabolite thereof, or a pharmaceutically acceptable salt thereof. An even more preferred selective serotonin re-uptake inhibitor is citalopram and is administered in a dose ranging between 10 and 40 mg of the active ingredient.

According to a further preferred embodiment, the invention relates to the use of pipamperone, preferably in a dose ranging from 5 to 15 mg of the active ingredient per day, in combination with citalopram, in a dose ranging from 10 to 40 mg of active ingredient per day, for treating diseases or disorders with an underlying dysregulation of the emotional functionality.

Preferred nor-epinephrine re-uptake inhibitors are chosen from the group consisting of, but not limited to tandamine, pirandamine, ciclazindol, fluparoxan, lortalamine, talsupram, talopram, prindamine, nomifensine, viloxazine, tomoxetine, duloxetine, venlafaxine, milnacipran and reboxetine, or a pro-drug or an active metabolite thereof, or a pharmaceutically acceptable salt thereof.

Preferred neuroleptic agents are chosen from the group consisting of, but not limited to chlorpromazine, haloperidol, perphenazine, thioridazine, mesoridazine, trifluoperazine, fluphenazine, clozapine, olanzapine, risperidone, paliperidone, ziprasidone, quetiapine, sertindole, aripiprazole, sonepiprazole, blonanserin, iloperidone, perospirone, raclopride, zotepine, DU-127090, ORG-5222, SM-13496, amisulpride, CP-361428, Lu 35-138, balaperidone, S-18327, WAY-135452, eplivanserin, E-5842, SR-31742, NE-100, osanetant, SR-141716, SR-48692, BSF-201640, BSF-190555, LAX-101a, sarizotan, CX-691 and SB-271046, or a pro-drug or an active metabolite thereof, or a pharmaceutically acceptable salt thereof.

Preferred NK1 antagonists are chosen from the group consisting of, but not limited to, MK-0869, GW597599, GW679769, GW823296, Compound A, NKP608, CP-96,345 (cis-3-(2-methoxybenzyl-amino-2-benzhydrylquinuclidine), CP-122721, CP-99994, GR-82334 (D-Pro9-[Spiro-y-lactam]-Leu10,Trp11)-Physalaemin(1-11)), R673, TAK-637, RPR100893 (perhydroisoindolol), RP-67580, LY303870, SR-140333 and trans-4-hydroxy-1-(1H-indol-3-ylcarbonyl)-L-prolyl-N-methyl-N-(phenylmethyl)-L-tyrosineamide (a derivative of FK888), or a pro-drug or an active metabolite thereof, or a pharmaceutically acceptable salt thereof. A more preferred NK1 antagonist is MK-0869 and is administered in a dose ranging between 10 and 160 mg of the active ingredient; even more preferred in a dose of 80 mg of the active ingredient.

According to a further preferred embodiment, the invention relates to the use of pipamperone, preferably in a dose ranging from 5 to 15 mg of the active ingredient per day, in combination with MK-0869, in a dose ranging from 10 to 160 mg of active ingredient per day, for treating diseases or disorders with an underlying dysregulation of the emotional functionality.

A still other group of pharmaceutical compounds whose effects are augmented or where the onset of the effect is fastened upon simultaneous or fore-going treatment with a selective 5-HT2A and D4 antagonist, inverse agonist or partial agonist, for instance pipamperone, are COX-2 inhibitors, used for treating or alleviating musculoskeletal diseases or disorders, such as rheumatoid arthritis, osteoarthritis or ankylosing spondylitis; or for the management of acute pain or for primary treatment of dysmenorrhea. Preferred COX-2 inhibitors are chosen from the group consisting of, but not limited to, celecoxib, rofecoxib, meloxicam, piroxicam, deracoxib, parecoxib, valdecoxib, etoricoxib, a chromene derivative, a chroman derivative, N-(2-cyclohexyloxynitrophenyl)-methane sulfonamide, COX189, ABT963 and JTE-522, or a pro-drug or an active metabolite thereof, or a pharmaceutically acceptable salt thereof.

COX-2 inhibition works through a direct neuronal mechanism; COX-2 enzyme does not kill the neurons through inflammation, but by oxidizing other molecules in the dopamine-producing cells. The oxidized molecules then react with and damage other components of the cell. Excessive damage can kill the cell. They are more familiar in arthritis, but the enzymes produce inflammation in all damaged tissues, including the brain. The present inventors found that inflammation might be a critical process in neurodegenerative diseases, and that oxidative damage might be responsible for killing of neurons in Parkinson's disease. Therefore, treatment of neurodegenerative diseases with COX-2 inhibitors may reduce aberrant and lethal oxidation in brain cells, thereby at least alleviating or ameliorating the disease.

It should be clear, given the general applicable character of the invention, that the herein presented list of other pharmaceutical compounds which effect is modulated, for instance augmented, by the selective 5-HT2A and D4 antagonists, inverse agonists or partial agonists of the present invention, and, accordingly the diseases or disorders which can be treated, is very brief and is should be clear that the invention should not be restricted to the ones exemplified herein.

It should be clear that the compounds and compositions described herein are useful for treating any patient in need thereof. As used herein the term "patient" is not restricted to humans but also to other mammals, for instance domestic animals which may also suffer from any form of a mental disease or disorder described herein.

The contents of all references cited herein are incorporated by reference into the present text.

The invention, now being generally described, will be more readily understood by reference to the following tables and examples, which are included merely for purposes of illustration of certain aspects and embodiments of the present invention and are not intended to limit the invention.

### Examples

### Example 1: Measuring pKi values of test compounds

In Table 1, the pKi values of test compounds are given for each of the dopamine receptors, 5HT receptors, adrenergic receptors and the histamine1 receptor. The affinity of test compounds for the respective receptors has been performed according to conventional procedures known in the art.

An indication "0" means that no affinity has been measured between the test compound and the receptor.

The columns displaying the pKi values for the D4 and the 5-HT2A receptor are filled with dark grey. pKi values between 8 and 9 and higher than 9 are represented by light grey shaded boxes.

### Example 2: Foregoing pipamperon-citalopram treatment in mayor depressive disorder: a placebo and active controlled period finding clinical trial

Table 2 represents the set-up of a clinical trial comprising for treatment groups:
Group Plc - Active / Day 0 represents the group receiving 10 mg citalopram, twice a day, starting the first day (Day 0) of active treatment in the clinical trial. This administration regime is also indicated as the mono therapy.
Group Pip - Active / Day 0 represents the group receiving a combination of 4 mg pipamperon and 10 mg citalopram, twice a day, starting the first day (Day 0) of active treatment in the clinical trial. This administration regime is also indicated as the non-foregoing combo therapy.
Group Pip - Active / Day 4 represents the group receiving 4 mg pipamperon, twice a day, starting the first day (Day 0) of active treatment in the clinical trial, followed by a combination of 4 mg pipamperon and 10 mg citalopram, twice a day, starting the fifth (Day 4) day of active treatment in the clinical trial. This administration regime is also indicated as the foregoing therapy with combination therapy starting after 4 days of active treatment.
Group Pip - Active / Day 7 represents the group receiving 4 mg pipamperon, twice a day, starting the first day (Day 0) of active treatment in the clinical trial, followed by a combination of 4 mg pipamperon and 10 mg citalopram, twice a day, starting the eight (Day 7) day of active treatment in the clinical trial. This administration regime is also indicated as the foregoing therapy with combination therapy starting after 7 days of active treatment.
All subjects also undergo a placebo (PLC) run-in therapy, administered during a period of about 7 days before the active treatment starts.
During daily (D), weekly (W) or monthly (M) visits, several parameters are measured.

Under NECT is to be understood: Neuronal E-clinical Trial = Vesalius Expert development for this trial which includes the bottom-up measurement of:
- In- and exclusion-criteria
- Functional status evaluation
- Medical history
- (Pre-)treatment signs & symptoms
- DSM-IV rules for diagnosis & efficacy
- HDRS-28 (Hamilton Depression Rating Scale - 28 items)
- Medical resource utilisation
- Pre-trial & Concomittant medication
- Drug administration
- (Serious) Adverse events
- Admission to the acute and extension phase of treatment
- Right flow of the trial

### Example 3: Foregoing pipamperon-pergolide treatment in Parkinson Disease: a placebo and active controlled period finding clinical trial

Table 3 represents the set-up of a clinical trial comprising for treatment groups:
Group Plc - Active / Day 0 represents the group receiving 1.5 mg pergolide, twice a day, starting the first day (Day 0) of active treatment in the clinical trial. This administration regime is also indicated as the mono therapy.
Group Pip - Active / Day 0 represents the group receiving a combination of 4 mg pipamperon and 1.5 mg pergolide, twice a day, starting the first day (Day 0) of active treatment in the clinical trial. This administration regime is also indicated as the non-foregoing combo therapy.
Group Pip - Active / Day 4 represents the group receiving 4 mg pipamperon, twice a day, starting the first day (Day 0) of active treatment in the clinical trial, followed by a combination of 4 mg pipamperon and 1.5 mg pergolide, twice a day, starting the fifth (Day 4) day of active treatment in the clinical trial. This administration regime is also indicated as the foregoing therapy with combination therapy starting after 4 days of active treatment.
Group Pip - Active / Day 7 represents the group receiving 4 mg pipamperon, twice a day, starting the first day (Day 0) of active treatment in the clinical trial, followed by a combination of 4 mg pipamperon and 1.5 mg pergolide, twice a day, starting the eight (Day 7) day of active treatment in the clinical trial. This administration regime is also indicated as the foregoing therapy with combination therapy starting after 7 days of active treatment.
All subjects also undergo a placebo (PLC) run-in therapy, administered during a period of about 7 days before the active treatment starts.
During daily (D), weekly (W) or monthly (M) visits, several parameters are measured.

Under NECT is to be understood: Neuronal E-clinical Trial = Vesalius Expert development for this trial which includes the bottom-up measurement of:
- In- and exclusion-criteria
- Functional status evaluation
- Medical history
- (Pre-)treatment signs & symptoms
- ICD-10 rules for diagnosis & efficacy
- UPDRS measurement in Parkinson's disease
- Medical resource utilization
- Pre-trial & Concomitant medication
- Drug administration
- (Serious) Adverse events
- Admission to the acute and extension phase of treatment
- Right flow of the trial

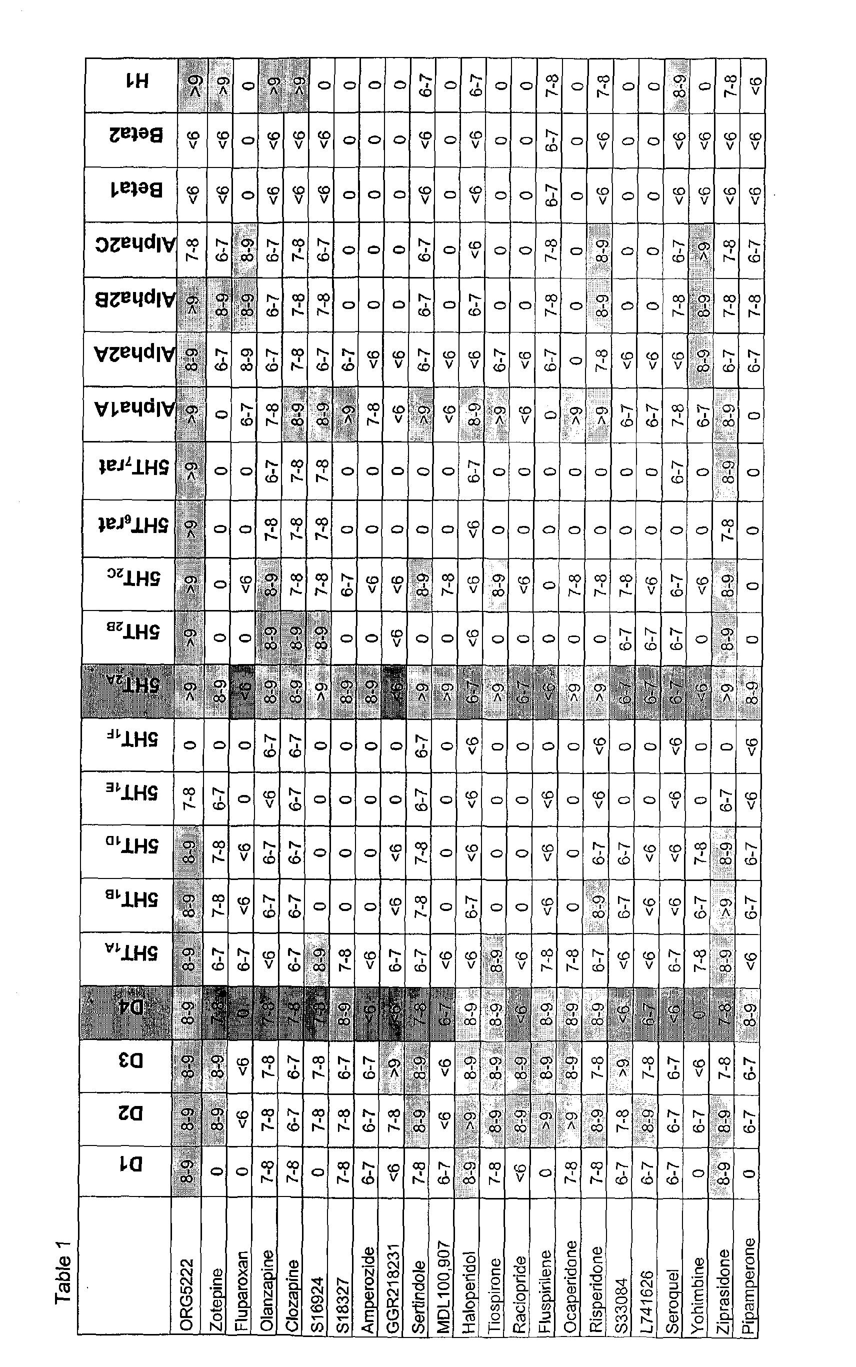

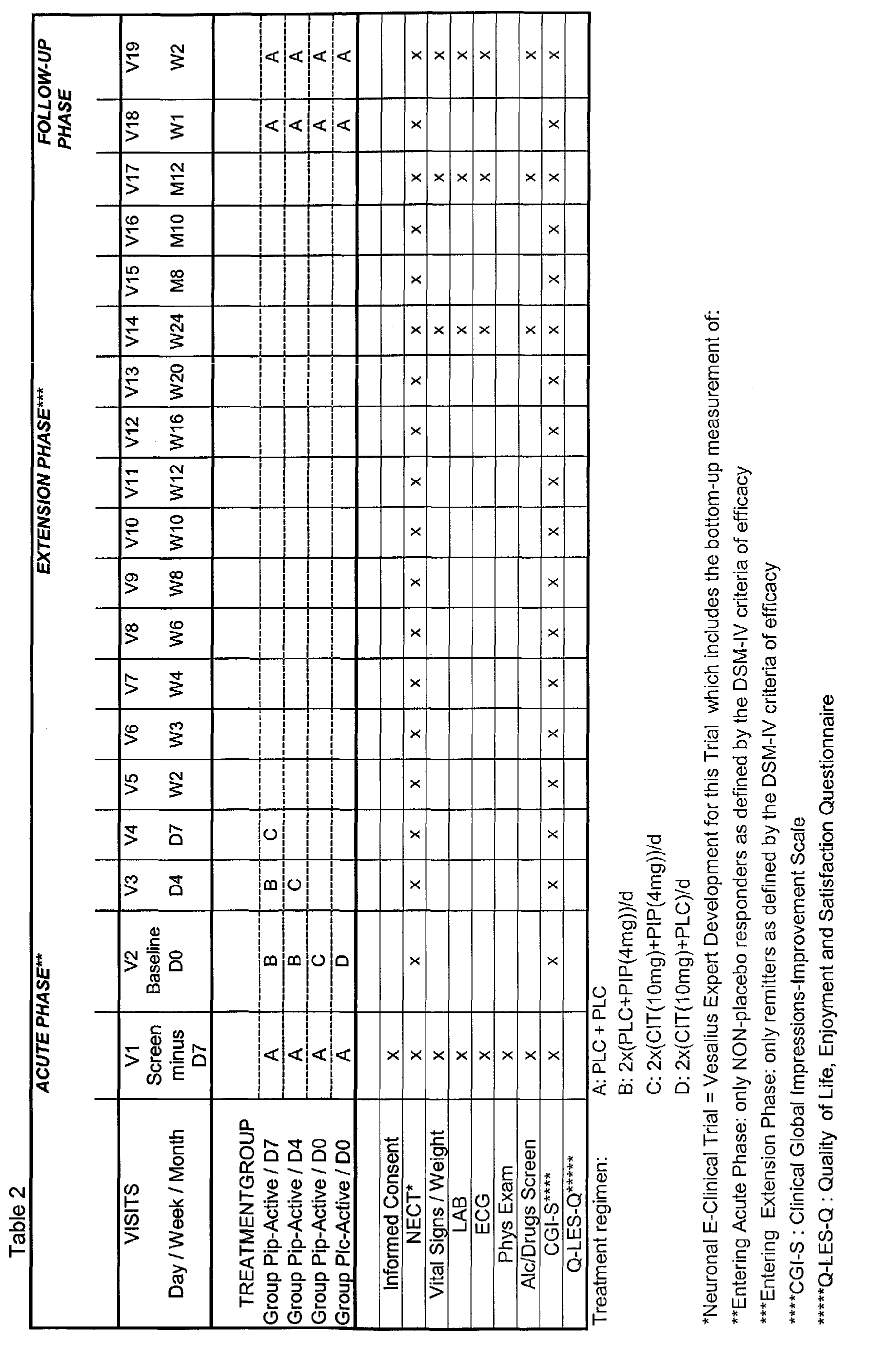

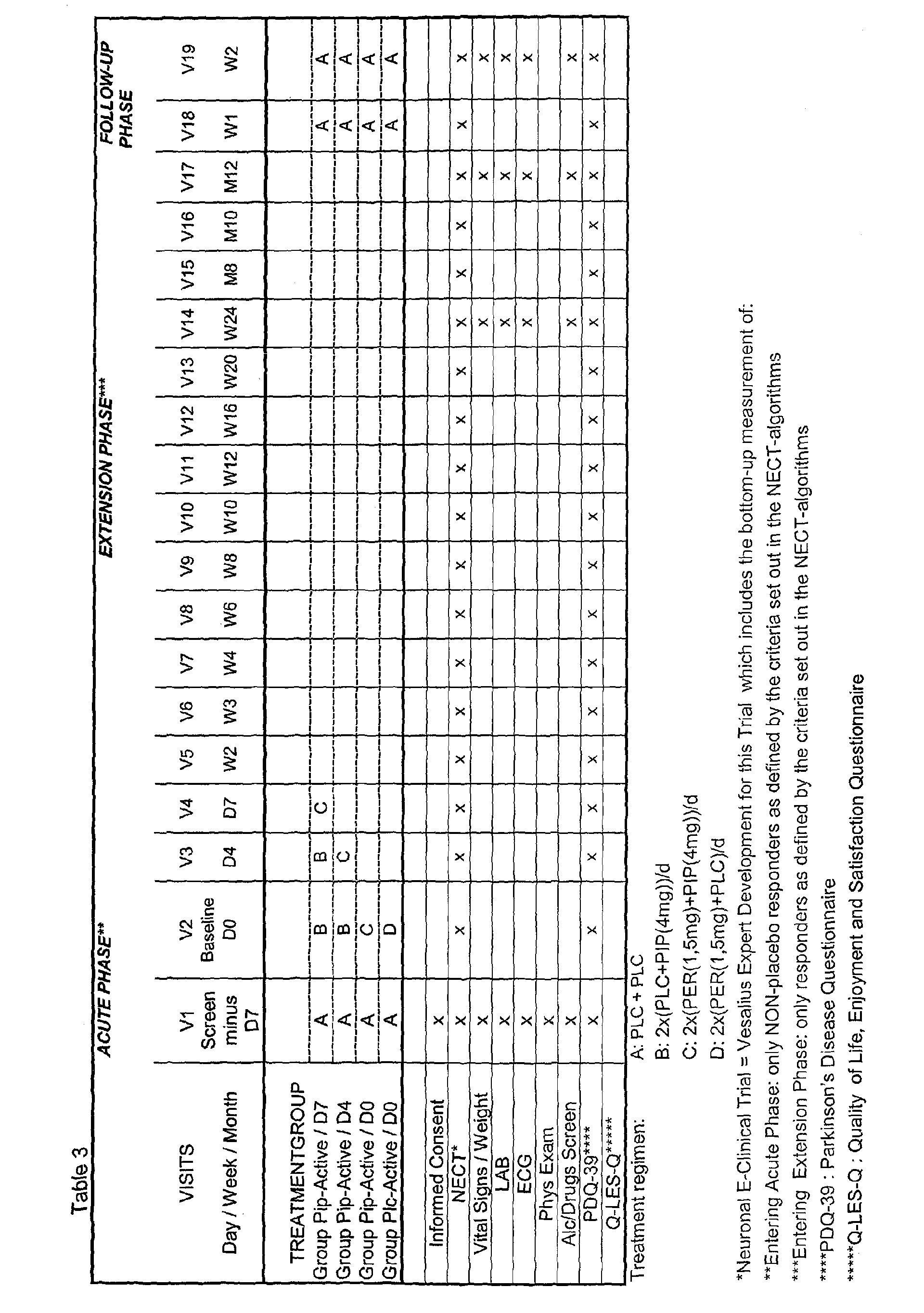

## Claims

1. Use of a first compound for the preparation of a medicament for treating neurodegenerative diseases or disorders, **characterized in that** said compound is administered simultaneously with, separate from or prior to the administration of a second compound to augment the therapeutic effect or to provide a faster onset of the therapeutic effect of said second compound, further **characterized in that** said first compound has (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors.

2. Use according to claim 1, wherein said first compound is pipamperone.

3. Use according to claim 2, wherein said first compound is to be administered to a patient in a dose ranging between 5 and 15 mg of the active ingredient.

4. Use according to any of claims 1 to 3, wherein said neurodegenerative disease or disorder is Parkinson Disease.

5. Use according to any of claims 1 to 4, wherein said first compound is to be administered daily at least one day before administering said second compound.

6. Use according to any of claims 1 to 5, wherein said second compound is a dopamine receptor agonist.

7. Use according to claim 6, wherein said dopamine receptor agonist is chosen from the group consisting of amantadine, bromocriptine, cabergoline lisuride, pergolide, ropinirole and pramipexole, or a pro-drug or an active metabolite thereof, or a pharmaceutically acceptable salt thereof.

8. Use according to claim 7, wherein said dopamine receptor agonist is pergolide and is to be administered in a dose ranging between 0.5 and 10 mg of the active ingredient.

9. A pharmaceutical composition comprising
(a) a compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors and
(b) a dopamine receptor agonist, as a combined preparation for simultaneous, separate or sequential use for treating a neurodegenerative disease or disorder such as Parkinson Disease.

10. Use according to any of claims 1 to 5, wherein said second compound is levodopa associated with a decarboxylase inhibitor.

11. Use according to claim 10, wherein said levodopa/decarboxylase-inhibitor is chosen from the group consisting of levodopa/carbidopa and levodopa/benserazide, or a pro-drug or an active metabolite thereof, or a pharmaceutically acceptable salt thereof.

12. Use according to claim 12, wherein said levodopa/decarboxylase-inhibitor is levodopa/carbidopa and is to be administered in a dose ranging between 2000 mg/ 200 mg and 100 mg/10 mg of the active ingredients.

13. A pharmaceutical composition comprising
(a) a compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors, and
(b) a levodopa associated with a decarboxylase inhibitor,
as a combined preparation for simultaneous, separate or sequential use for treating a neurodegenerative disease or disorder such as Parkinson Disease.

14. Use according to any of claims 1 to 5, wherein said second compound is a mono-amine oxidase B (MAO-B) inhibitor.

15. Use according to claim 14, wherein said second compound is selegilinehydrochloride or a pro-drug or an active metabolite thereof, or a pharmaceutically acceptable salt thereof.

16. Use according to claim 15, wherein said selegilinehydrochloride is to be administered in a dose ranging between 2 and 25 mg of the active ingredient.

17. A pharmaceutical composition comprising
(a) a compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors and
(b) a mono-amine oxidase B (MAO-B) inhibitor,
as a combined preparation for simultaneous, separate or sequential use for treating a neurodegenerative disease or disorder such as Parkinson Disease.

18. Use of a composition for the preparation of a medicament for treating a neurodegenerative disease or disorder, **characterized in that** said composition is administered simultaneously with, separate from or prior to the administration of a third compound to augment the therapeutic effect or to provide a faster onset of the therapeutic effect of said third compound, further **characterized in that** said composition comprises a first compound having (i) a selective affinity for the D4 receptor with a pKi value equal to or higher than 8 towards the D4 receptor and less than 8 towards other Dopamine receptors, and a second compound having (ii) a selective affinity for the 5-HT2A receptor with a pKi value equal to or higher than 8 towards the 5-HT2A receptor and less than 8 towards other 5HT receptors.

19. Use according to claim 18, wherein said neurodegenerative disease or disorder is Parkinson Disease.

20. Use according to claim 18 or 19, wherein said first compound is chosen from the group consisting of, pipamperone, fananserin, L-745,870, PNU-101387G and U-101387 or a pro-drug or a pharmaceutically acceptable salt thereof and wherein said second compound is chosen from the group comprising pipamperone, fananserin, ORG 5222, zotepine, olanzepine, clozapine, S16924, S18327, amperozide, serindole, MDL 100.907, tiospirone, fluspirilene, ocaperidone, risperidone, paliperidone and ziprasidone or a pro-drug or an active metabolite thereof, or a pharmaceutically acceptable salt thereof.

21. Use according to any of claims 18 to 20, wherein said composition is to be administered to a patient in a dose ranging between 0.5 µg and 2000 mg for each of the active ingredients.

22. Use according to any of claims 18 to 20, wherein said third compound is a dopamine receptor agonist.

23. Use according to claim 22, wherein said dopamine receptor agonist is chosen from the group consisting of amantadine, bromocriptine, cabergoline lisuride, pergolide, ropinirole and pramipexole, or a pro-drug or an active metabolite thereof, or a pharmaceutically acceptable salt thereof.

24. Use according to claim 23, wherein said dopamine receptor agonist is pergolide and is to be administered in a dose ranging between 0.5 and 10 mg of the active ingredient.

25. A pharmaceutical composition comprising
(a) a compound having a selective affinity for the Dopamine-4 (D4) receptor with a pKi value equal to or higher than 8 towards the D4 receptor and less than 8 towards other Dopamine receptors,
(b) a compound having a selective affinity for the 5-HT2A receptor with a pKi value equal to or higher than 8 towards the 5-HT2A receptor and less than 8 towards other 5HT receptors, and
(c) a dopamine receptor agonist,
as a combined preparation for simultaneous, separate or sequential use for treating a neurodegenerative disease or disorder such as Parkinson Disease.

26. Use according to any of claims 18 to 20, wherein said third compound is levodopa associated with a decarboxylase inhibitor.

27. Use according to claim 26 wherein said levodopa/decarboxylase-inhibitor is chosen from the group comprising levodopa/carbidopa, levodopa/benserazide, or a pro-drug or an active metabolite thereof, or a pharmaceutically acceptable salt thereof.

28. Use according to claim 27, wherein said levodopa/decarboxylase-inhibitor is levodopa/carbidopa and is to be administered in a dose ranging between 2000 mg/ 200 mg and 100 mg/ 10 mg of the active ingredients.

29. A pharmaceutical composition comprising
(a) a compound having a selective affinity for the Dopamine-4 (D4) receptor with a pKi value equal to or higher than 8 towards the D4 receptor and less than 8 towards other Dopamine receptors, and
(b) a compound having a selective affinity for the 5-HT2A receptor with a pKi value equal to or higher than 8 towards the 5-HT2A receptor and less than 8 towards other 5HT receptors, and
(c) levodopa associated with a decarboxylase inhibitor,
as a combined preparation for simultaneous, separate or sequential use for treating a neurodegenerative disease or disorder such as Parkinson Disease.

30. Use according to any of claims 18 to 20, wherein said third compound is a mono-amine oxidase B (MAO-B) inhibitor.

31. Use according to claim 30, wherein said mono-amine oxidase B (MAO-B) inhibitor is selegelinehydrochloride or a pro-drug or an active metabolite thereof, or a pharmaceutically acceptable salt thereof.

32. Use according to claim 31, wherein selegilinehydrochloride is to be administered in a dose ranging between 2 and 25 mg of the active ingredient.

33. A pharmaceutical composition comprising
(a) a compound having a selective affinity for the Dopamine-4 (D4) receptor with a pKi value equal to or higher than 8 towards the D4 receptor and less than 8 towards other Dopamine receptors, and
(b) a compound having selective affinity for the 5-HT2A receptor with a pKi value equal to or higher than 8 towards the 5-HT2A receptor and less than 8 towards other 5HT receptors. and
(c) a mono-amine oxidase B (MAO-B) inhibitor,
as a combined preparation for simultaneous, separate or sequential use for treating a neurodegenerative disease or disorder such as Parkinson Disease.

34. Use of a compound as defined in any of claims 1 to 3, or of a composition as defined in claim 18, for the preparation of a medicament for treating a cognitive disease or disorder, **characterized in that** said compound or composition is administered simultaneously with, separate from or sequential to a cholinesterase inhibitor to augment the therapeutic effect or to provide a faster onset of the therapeutic effect of said cholinesterase inhibitor.

35. Use according to claim 34, wherein said disease or disorder is selected from the group consisting of delirium; dementia, such as Alzheimer Disease, substance-induced persisting dementia, vascular dementia, dementia due to a general medical condition chosen from the group comprising HIV disease, head trauma, Parkinson Disease, Huntington Disease, Pick Disease and Creutzfeldt-Jacob Disease; amnestic disorders due to a general medical condition or a substance-induced persisting amnestic disorder; mild cognitive impairment disorder; and other cognitive disorders.

36. Use according to claim 34 or 35, wherein said cholinesterase inhibitor is chosen from the group consisting of donepezil, ENA-713, galantamine, memantine and tacrine, or a pro-drug or an active metabolite thereof, or a pharmaceutically acceptable salt thereof.

37. Use according to claim 36, wherein said cholinesterase inhibitor is galantamine and is to be administered in a dose ranging between 5 and 50 mg of the active ingredient.

38. A pharmaceutical composition comprising
(a) a compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors, and
(b) a cholinesterase inhibitor
as a combined preparation for simultaneous, separate or sequential use for treating a cognitive disease or disorder.

39. A pharmaceutical composition comprising
(a) a compound having a selective affinity for the Dopamine-4 (D4) receptor with a pKi value equal to or higher than 8 towards the D4 receptor and less than 8 towards other Dopamine receptors, and
(b) a compound having a selective affinity for the 5-HT2A receptor with a pKi value equal to or higher than 8 towards the 5-HT2A receptor and less than 8 towards other 5HT receptors, and
(c) a cholinesterase inhibitor ,
as a combined preparation for simultaneous, separate or sequential use for treating a cognitive disease or disorder.

40. A pharmaceutical composition according to any of claims 9, 13, 17 or 38 wherein said compound having (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5HT receptors, is pipamperone and is present in the composition in a dose ranging between 5 and 15 mg of active ingredient, expressed as the daily dose to be administered to a patient in need thereof.
